# EUROPEAN PATENT APPLICATION

(11) **EP 3 901 117 A1**
(43) Date of publication of application: **27.10.2021**
(21) Application number: 19817824.6
(22) Date of filing: 31.10.2019
(51) Int. Cl.: C05D 9/02, C05F 11/00, C05G 5/10, C05G 5/23, A01N 63/30

(54) **LYOPHILIZED FERTILIZER COMPOSITION COMPRISING IRON SIDEROPHORE CHELATES, LYOPHILIZED COMPOSITION COMPRISING SIDEROPHORES, THEIR PROCESSES OF OBTENTION AND USES TO TREAT PLANTS**

(30) Priority: 17.12.2018 PT 2018115218
(71) Applicant: Universidade do Porto, 4099-002 Porto (PT); Instituto Superior de Engenharia do Porto, 4249-015 Porto (PT); Universidad Autónoma De Madrid (UAM), 28049 Madrid (ES)
(72) Inventor: MONTEIRO SOARES, Helena Maria Vieira, 4099-002 PORTO (PT); HENRIQUES FERREIRA, Carlos Miguel, 4099-002 PORTO (PT); VALENTE SOARES, Eduardo Jorge, 4099-002 PORTO (PT); LUCENA MAROTTA, Juan José, MADRID (ES); LOPÉZ RAYO, Sandra, MADRID (ES)
(74) Representative: Monteiro Alves, Inês
(86) International application number: PCT/PT2019/050041
(87) International publication number: WO 2020/130862

(57) **Abstract**

Lyophilized fertilizer composition comprising iron siderophore chelates and a stabilizing agent and a lyophilized composition comprising siderophores and a stabilizing agent, wherein the stabilizing agent is starch. The invention further refers to processes of obtaining a lyophilized fertilizer composition comprising a step of synthesis of at least one siderophore compound by means of biochemical transformation executed by at least one microorganism and to the use of the compositions for treating cultivated plants, for example in conditions of low iron availability, where the compositions have the ability of correcting chlorosis in plants, not presenting environmental risks. The lyophilized fertilizer composition and the lyophilized composition have high storage stability.

## Description

### TECHNICAL FIELD OF THE INVENTION

The present invention refers to a lyophilized fertilizer composition comprising iron siderophore chelates and a stabilizing agent and their processes of obtention.

The present invention also refers to a lyophilized composition comprising siderophores and a stabilizing agent and their processes of obtention.

The present invention further comprises the use of a lyophilized fertilizer composition comprising iron siderophore chelates and a stabilizing agent and of the lyophilized composition comprising siderophores and a stabilizing agent for treating plants, particularly in plants cultivated under conditions of low iron availability.

### STATE OF THE ART

Iron is an essential nutrient for the development of plants in conjunction with other macro and micronutrients. Although iron is not present in chlorophyll, this element is essential for its synthesis and for the process of the photosynthetic system.

Iron deficiency is one of the main causes of chlorosis in plants. The chlorotic plants present an underdeveloped state, producing less flowers and fruits. Chlorosis presents a myriad of symptoms, and a plant with this deficiency may be characterized by presenting young yellowish leaves, resulting from the inefficient production of chlorophyll. The role of iron as a nutrient and its relation with chlorosis is referred in the works of Barker, A. V., Stratton, M.L. (Iron, In: "Handbook of Plant Nutrition" CRC Press, Boca Raton, Florida, EUA, 2015, pp. 399-426) and in Broadley et al. ("Function of Nutrients, in: Marschner's Mineral Nutrition of Higher Plants", Elsevier, 2012, pp. 191-248) and in Lucena, J.J ("Effects of bicarbonate, nitrate and other environmental factors on iron deficiency chlorosis. A review", J. Plant Nutr. 23, 1591-1606, 2000).

The low availability of iron results from its chemical nature, its low solubility and its slow dissolution kinetics, which limit the iron absorption by the plants, particularly in calcareous soils. Chlorosis is a problem in agriculture, since this deficiency may result in a significant reduction of the harvest yields. Chlorotic plants produce less biomass, flowers and fruits, and, at the limit, the complete loss of the plantation may occur, as referred in Guerinot, M.L., Yi, Y. ("Iron: Nutritious, Noxious, and Not Readily Available", Plant Physiol. 104, 815-820, 1994).

Iron deficiency constitutes a global problem, particularly severe in calcareous soils. It is estimated that the calcareous soils cover about 30% of the earth surface in the world, as referred in Cianzio et al. ("Genomic Resources of Agronomic Crops, in: Iron Nutrition in Plants and Rhizospheric Microorganisms". Springer Netherlands, Dordrecht, pp. 449-466, 2006).

The challenge of reversing the chlorosis induced by the iron deficiency in agriculture and elevating the yields of the harvests has been the focus of great interest. For this purpose, synthetic compounds were developed, for example the aminopolycarboxylic acids (APCAs), which present an elevated iron complex stability. Although the aminopolycarboxylic acids are strong chelating agents, usually they are not biodegradable. Therefore, their persistence and consequent accumulation in aquatic systems is becoming a matter of concern, since the aminopolycarboxylic acids may also form complexes with toxic metals, retaining them, as referenced in Bucheli-Witschel, M., Egli, T. ("Environmental fate and microbial degradation of aminopolycarboxylic acids". FEMS Microbiol. Rev. 25, 69-106, 2001).

Therefore, the current use of synthetic chelates to prevent or correct the iron deficiency in plant cultivations is not satisfactory, since the referred synthetic iron chelates are related to environmental problems.

Thus, the search of iron chelates, environmentally friendly, with adequate properties to be used as fertilizers (to provide iron for the development of plants) has become a big challenge.

Within the context of a large number of compounds that are considered, the siderophores are an interesting study object, since they are effective iron chelates and have the advantage of being more biodegradable than the synthetic chelates (aminopolycarboxylic acids), as referenced in Fazary et. Al. ("Biodegradable siderophores: survey on their production, chelating and complexing properties". Rev. Inorg. Chem. 36, 153-181, 2016).

One problem of the state of the art is related to the fact that the application of purified siderophores or the direct application of microbial cultures containing siderophores to fertilize soils is not feasible, due to the large volumes that are required in the handling thereof, causing transport and storage problems. On the other hand, another problem of the state of the art is related to the fact that the siderophores are natural products and potentially biodegradable, whereby a long storage period in aqueous form, would lead to the degradation of the siderophores with a consequent decrease in their concentration.

Therefore, there exists a great interest and need to create a fertilizer composition that is effective in fighting and/or preventing chlorosis in plants, where the fertilizer is biodegradable and environmentally friendly. It is a further demand of the state of the art that the active ingredient of the fertilizer be stable during long storage periods. Another need of the state of the art is that the volumes of fertilizer to be handled during transport and treatment of the plants are reduced.

### BRIEF DESCRIPTION OF THE DRAWINGS

With the purpose of promoting an understanding of the principles in accordance with the embodiments of the present invention, reference will be made to the embodiments illustrated in the figures and to the language used to describe the same. Anyway, it must be understood that there is no intention of limiting the scope of the present invention to the contents of the figures. Any alterations or later changes of the inventive features illustrated herein and any additional application of the principles and embodiments of the invention shown, which would occur normally for one skilled in the art when reading this description, are considered as being within the scope of the claimed invention.
Figure 1 - illustrates one embodiment of the process for obtaining a lyophilized fertilizer composition comprising iron siderophore chelates and a stabilizing agent;
Figure 2 - illustrates another embodiment of the process for obtaining a lyophilized fertilizer composition comprising iron siderophore chelates and a stabilizing agent;
Figure 3 - illustrates an embodiment of the process for obtaining a lyophilized composition comprising siderophores and a stabilizing agent;
Figure 4 - illustrates another embodiment of the process for obtaining a lyophilized composition comprising siderophores and a stabilizing agent;
Figure 5 - illustrates a growth curve of a strain of *Azotobacter vinelandii;*
Figure 6 - illustrates the production of siderophores during the growth of a strain of *Azotobacter vinelandii;*
Figure 7 - illustrates comparative tests of the developments of the cultivation of plants with treatments with an iron chelate;
Figure 8 - illustrates comparative tests of the developments of the cultivation of plants with treatments with an iron chelate as regards the levels of chlorophyll quantified in the second leaf stage;
Figure 9 - illustrates comparative tests of the developments of the cultivation of plants with treatments with an iron chelate as regards the levels of chlorophyll quantified at the end of 21 days, between the second and the fifth leaf stages;
Figure 10 - illustrates comparative tests of the developments of the cultivation of plants with treatments with an iron chelate as regards the iron content of the plant.

Legends of the reference numbers:
(1) inoculum of at least one microorganism;
(2) propagation of the microorganisms in an iron enriched nutrient medium;
(3) separation, with eventual washing, of the microorganisms;
(4) isolated microorganisms;
(5) filtrate originated from the iron enriched medium;
(6) propagation of microorganisms in an iron deficient nutrient medium (synthesis of at least one siderophore compound by means of the biochemical transformation executed by at least one microorganism);
(7) biotransformed medium, originating from the synthesis of at least one siderophore compound by means of a biochemical transformation executed by at least one microorganism, comprising at least one siderophore compound;
(8) addition of an iron source;
(9) pH adjustment;
(10) separation of insoluble solids;
(11) insoluble solids;
(12) aqueous solution comprising iron siderophore chelates;
(13) addition of stabilizing agent;
(14) lyophilization;
(15) lyophilized fertilizer composition comprising iron siderophore chelates and a stabilizing agent; and
(16) lyophilized composition comprising siderophores and a stabilizing agent.

### DETAILED DESCRIPTION OF THE INVENTION

In a first aspect, the present invention refers to a process for obtaining a lyophilized fertilizer composition including iron siderophore chelates comprising the following steps:
**a)** Synthesis of at least one siderophore compound by means of a biochemical transformation executed by at least one microorganism, forming a biotransformed medium, comprising at least one siderophore compound;
**b)** Addition of an iron source to the biotransformed medium, comprising at least one siderophore compound, forming an aqueous solution comprising iron siderophore chelates;
**c)** Addition of a stabilizing agent to the aqueous solution comprising iron siderophore chelates, forming a mixture comprising iron siderophore chelates wherein the stabilizing agent is starch, for example cornstarch;
**d)** Lyophilization of a mixture comprising iron siderophore chelates and a stabilizing agent, forming a lyophilized fertilizer composition comprising iron siderophore chelates and a stabilizing agent.

The present invention, in a second aspect, refers to a process for obtaining a lyophilized fertilizer composition including iron siderophore chelates comprising the following steps:
a) Synthesis of at least one siderophore compound by means of a biochemical transformation executed by at least one microorganism, forming a biotransformed medium comprising at least one siderophore compound;
b) Addition of an iron source to the biotransformed medium comprising at least one siderophore compound, forming an aqueous solution comprising iron siderophore chelates;
c) Lyophilization of the aqueous solution comprising iron siderophore chelates, forming a lyophilized fertilizer composition comprising iron siderophore chelates;
d) Addition of a stabilizing agent to the lyophilized fertilizer composition comprising iron siderophore chelates, forming a lyophilized fertilizer composition comprising iron siderophore chelates and a stabilizing agent, wherein the stabilizing agent is starch, for example cornstarch.

The present invention, in a third aspect, refers to a process for obtaining a lyophilized composition including siderophores comprising the following steps:
a) Synthesis of at least one siderophore compound by means of a biochemical transformation executed by at least one microorganism, forming a biotransformed medium comprising at least one siderophore compound;
b) Addition of a stabilizing agent to the biotransformed medium comprising at least one siderophore compound, forming a mixture comprising siderophores and a stabilizing agent wherein the stabilizing agent is starch, for example cornstarch;
c) Lyophilization of a mixture comprising siderophores and a stabilizing agent, forming a lyophilized composition comprising siderophores and a stabilizing agent.

The present invention, in a fourth aspect, refers to a process for obtaining a lyophilized composition including siderophores comprising the following steps:
a) Synthesis of at least one siderophore compound by means of a biochemical transformation executed by at least one microorganism, forming a biotransformed medium comprising at least one siderophore compound;
b) Lyophilization of the biotransformed medium comprising at least one siderophore compound, forming a lyophilized composition comprising siderophores;
c) Addition of a stabilizing agent to the lyophilized composition comprising siderophores, forming a lyophilized composition comprising siderophores and a stabilizing agent, wherein the stabilizing agent is starch, for example cornstarch.

The present invention, in a fifth aspect, refers to a lyophilized fertilizer composition comprising iron siderophore chelates and a stabilizing agent, wherein the stabilizing agent is starch, for example cornstarch.

The present invention, in a sixth aspect, refers to a lyophilized composition comprising siderophores and a stabilizing agent, wherein the stabilizing agent is starch, for example cornstarch.

The present invention, in a seventh aspect, refers to the use of a lyophilized fertilizer composition including iron siderophore chelates, according to the present invention, for treating plants.

The present invention, in an eighth aspect, refers to the use of a lyophilized composition including siderophores, according to the present invention, for treating plants.

The lyophilized fertilizer composition of the present invention is able of correcting chlorosis in plants, induced by iron deficiency. Additionally, the fertilizer composition of the present invention does not present environmental risks, when compared to the synthetic iron chelates, since the fertilizer composition of the present invention is produced by means of a biotechnological process. Further, the fertilizer composition of the present invention is capable of potentially solving the problems resulting from chlorosis for different kind of plantations. The fertilizer compositions of the present invention are highly stable in terms of storage. The lyophilized fertilizer compositions of the present invention present smaller volumes for handling, thus reducing costs relating to transport, storage and application. In a particularly beneficial manner, after an adequate rehydration process, the lyophilized fertilizer compositions of the present invention may be directly applied in calcareous soils, in hydroponic plants or by means of foliar application.

In one of the embodiments of the present invention, the synthesis step of at least one siderophore compound, by means of a biochemical transformation executed by at least one microorganism, comprises:
a first propagation of microorganisms in an iron enriched nutrient medium; and
a second propagation of the microorganisms in an iron deficient nutrient medium.

In the first propagation step of the microorganisms in an iron-enriched nutrient medium, no significant amount of siderophores are synthetised by the microorganisms involved.

The inoculation of microorganisms in an iron enriched medium has as objective the respective propagation without any limitation, ie, all the nutrients are present in the medium, in order not to limit the microbial growth. The iron is an important cofactor, necessary for the development of the microorganisms, being, for example, present in cytochromes, catalases and peroxidases.

The synthesis of siderophores occurs substantially in the second propagation of microorganisms in an iron deficient nutrient medium. In the preparation of the culture medium, which is used for the growth of microorganisms in a nutrient medium without iron, the iron source is not added. The inoculation with at least one microorganism, originating from the propagation of the microorganisms in an iron enriched nutrient medium, is prepared after the separation of the microorganisms in the first propagation of an iron enriched nutrient medium. It is understood by one skilled in the art that the inoculation with the separated microorganisms from the first propagation and subsequently inoculated in the second step of propagation may present residual amounts of iron. However, except for these residual quantities, no addition of iron whatsoever is made in the preparation of the nutrient medium used in the step of propagation of microorganisms in an iron deprived nutrient medium.

In the preferred embodiments of the present invention, after the synthesis of at least one siderophore compound by means of a biochemical transformation executed by at least one microorganism, that is, after the growth of the microorganisms in an iron deprived nutrient medium, the separation of the microorganisms from the biotransformed medium comprising at least one siderophore compound is carried out, wherein the biotransformed medium comprises the filtrate of the step of the second propagation of microorganisms in an iron deficient nutrient medium.

The separation of microorganisms is carried out so that the lyophilization step is conducted from an aqueous solution, in a more controlled manner, without other undesired components, eventually resulting from the microbial biomass, interfering in the lyophilized fertilizer composition desired at the end of the process.

In one of the embodiments of the present invention, in the step of addition of an iron source to the biotransformed medium comprising at least one siderophore compound, the pH adjustment is made in the range of from about 7 to about 9. In this step, the iron siderophore chelates are formed. In the preferred embodiments of the present invention, the iron source is at least one iron salt, which may present the iron cation in any of the valences corresponding to iron (II) or to the iron (III). It will be understood by one skilled in the art, that in conditions of presence of oxygen, at least one part of iron (II), eventually present, may be oxidized to iron (III). The iron salts that may be used in the present invention may be selected from nitrate, chloride and sulfate salts, for example Fe(NO₃)₃ and FeSO₄, although not limited to these. Alternatively, saturated aqueous solutions of iron salts may also be used as iron sources.

In another embodiment of the present invention, in the step of addition of an iron source to the biotransformed medium comprising at least one siderophore compound a separation of insoluble solids is carried out. The insoluble salts may comprise non-solubilized iron compounds. In the preferred embodiments of the present invention, the step of separation of insoluble solids, when necessary, is carried out after the pH adjustment step.

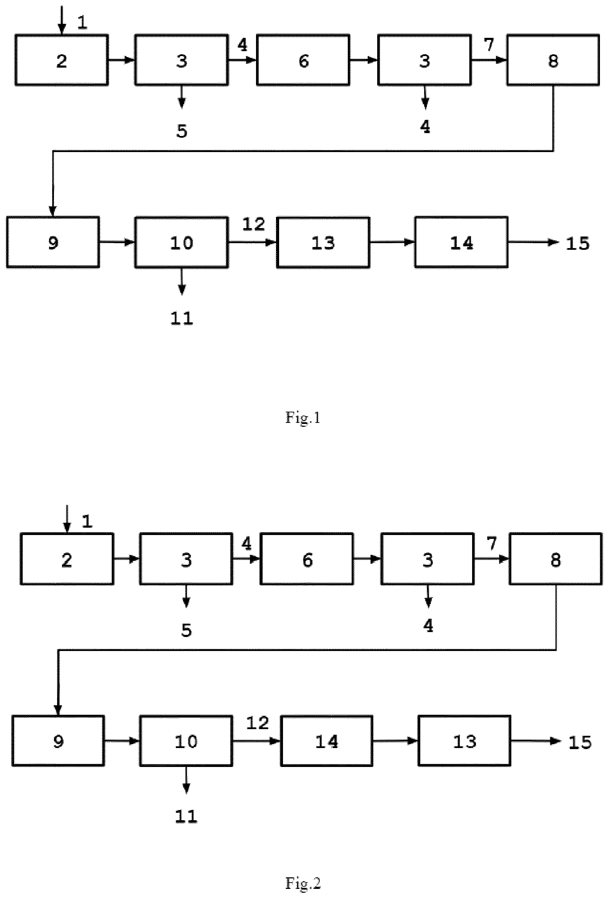

One preferred embodiment of the process for preparation of the lyophilized fertilizer composition according to the present invention is illustrated in Figure 1. Initially, the inoculation (1) and propagation of microorganisms in an iron enriched medium (2) of at least one microorganism capable of sinthetizing one or more siderophores from a nutrient medium are carried out. After the adequate growth of the microbial population, the separation of the microorganisms (3) is carried out, wherein the separation may be conducted by means of operations known to one skilled in the art, such as filtration or centrifugation. At least one part of the isolated microorganisms (4) is washed, inoculated and propagated in an iron deficient nutrient medium (6), whereby it is in this second propagation that the substantial synthesis of at least one siderophore compound occurs by means of a biochemical transformation executed by at least one microorganism. After growth, the microbial biomass is submitted to a separation step (3) from which the microorganisms (4) are isolated.

The biotransformed medium, originating from the synthesis of at least one siderophore compound by means of a biochemical transformation executed by at least one microorganism, comprising at least one siderophore compound (7) corresponds to the filtrate stream originating from the step of propagation of the microorganisms in an iron deficient nutrient medium (6).

It will be understood by one skilled in the art that the development of the step of propagation of microorganisms in an iron deficient medium (6) may be accompanied by means of analysis of the concentrations of siderophores in the filtrate stream, so as to decide to terminate the step of production of siderophores.

Subsequently, the addition step of an iron source (8) to the biotransformed medium is carried out, so, that at least one siderophore compound forms chelates with the added iron cations. After the addition of the iron source, the pH adjustment (9) is made in the range of from about 7 to about 9. Subsequently, the insoluble iron in the aqueous solution and other insoluble solids (11), eventually present, are submitted to a step of separation of insoluble solids (10), whereby this separation step may be comprised of the usual means to one skilled in the art, such as filtration, decantation or centrifugation.

After the formation of the aqueous solution comprising iron siderophore chelates (12), the step of the addition of the stabilizing agent (13) is carried out, in which the stabilizing agent is starch, for example cornstarch. Subsequently, the lyophilization step (14) is carried out, obtaining a lyophilized fertilizer composition comprising iron siderophore chelates and a stabilizing agent (15).

It will be understood by one skilled in the art that the usual techniques of lyophilization known to the state of the art will be used.

In one variant of the embodiment shown in Figure 1, as illustrated in Figure 2, in the process of obtaining a lyophilized fertilizer composition according to the present invention, the step of lyophilization (14) of the aqueous solution comprising iron siderophore chelates (12) may be carried out prior to the step of addition of the stabilizing agent (13).

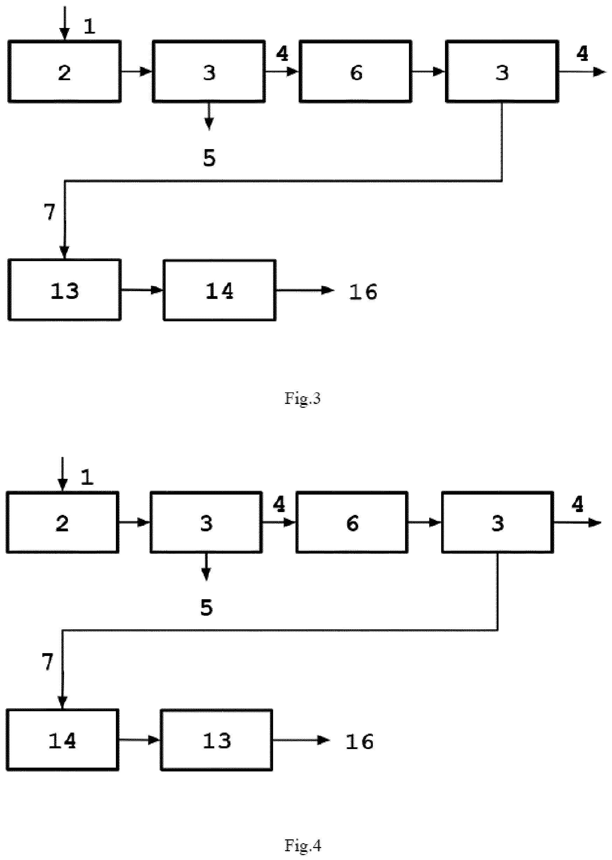

Another embodiment of the process of preparation of the lyophilized composition comprising siderophores and a stabilizing agent according to the present invention is illustrated in Figure 3. Initially, the inoculation (1) and the propagation of the microorganisms in an iron enriched nutrient medium (2) of at least one microorganism capable of synthetizing one or more siderophores from a nutrient medium is carried out. After the adequate growth of the microbial population, the separation of microoganisms (3) is carried out, wherein the separation may be conducted by means of operations known to one skilled in the art, such as filtration or centrifugation. At least one part of the isolated microorganisms (4) is subsequently inoculated and propagated in an iron deficient nutrient medium (6), whereby it is in this second propagation that the substantial synthesis of at least one siderophore compound by means of a biochemical transformation carried out by at least one microorganism takes place. After the desired growth of the microorganisms, the microbial mass is once again submitted to a separation step (3), wherein the microorganisms are isolated (4). The biotransformed medium, originating from the synthesis of at least one siderophore compound by means of a biochemical transformation executed by at least one microorganism, comprising at least one siderophore compound (7) corresponds to the filtrate stream resulting from the step of propagation of microorganisms in an iron deficient nutrient medium (6). After the formation of the biotransformed medium stream, the addition of the stabilizing agent (13) is made, wherein the stabilizing agent is starch, for example cornstarch. Subsequently, the lyophilization step (14) is carried out, obtaining a lyophilized composition comprising siderophores and a stabilizing agent (16).

In a variant of the embodiment illustrated in Figure 3, as illustrated in Figure 4, in the process of obtaining a lyophilized composition comprising siderophores and a stabilizing agent, according to the present invention, the lyophilization step (14) of the biotransformed medium (7) may be carried out prior to the step of addition of the stabilizing agent (13).

The microorganisms used to produce the siderophores may belong to one or more genus selected from *Azotobacter, Azospirillum, Bacillus, Enterobacter, Nocardia, Pantoea, Paracoccus, Pseudomonas, Rhizobium, Salinispora, Streptomyces* and *Vibrio.* Alternativaly, microorganisms belonging to risk group 1 may be used, wherein the microorganism presents low probability of causing diseases in Humans, as described in European Directive 2000/54/C or as referenced in the Committee on Biological Agents, according to the technical rulings for biological agents ("Technical Rules for Biological Agents (TRBA)-466", Committee on Biological Agents, 2015). It will be evident to one skilled in the art that microbial consortiums of selected genus may be used.

In the embodiments particulatly preferred in the present invention, microorganisms of the species *Azotobacter vinelandii, Bacillus megaterium, Bacillus subtilis, Pantoea allii, Pseudomonas fluorescens* and *Rhizobium radiobacter* are used. As referenced in Hamilton et al. ("Transcriptional Profiling of Nitrogen Fixation in Azotobacter vinelandii." J. Bacteriol. 193, 4477-4486, 2011) and in Knowles, C.J., Redfearn, E.R. ("Cytochrome and ubiquinone patterns during growth of Azotobacter vinelandii.", J. Bacteriol. 97, 756-60, 1969), the microorganism *Azotobacter vinelandii* has the ability to fixate atmospheric nitrogen, thus, in the preferred embodiments of the present invention using this microorganism, a nitrogen source deficient culture medium is used, as for example, a Burk medium.

In fact, a relatively small number of bacteria is able to fix the atmospheric nitrogen and converting it into ammonia, thus, the absence of a nitrogen source in the culture medium creates selective growth conditions. The growth of a strain of a microorganism in the absence of a nitrogen source has an extremely practical importance in the stage of biotechnological production of siderophores, since it is not likely that a strain *Azotobacter vinelandii,* for example, is contaminated by undesirable microoganisms. Furthermore, in the absence of a nitrogen source, a culture medium, such as the Burk medium, presents the advantage of lower preparation costs, since the medium may present solely mineral salts and glucose and/or other carbon source as substrate, which will result in a reduction of the costs of preparation of the lyophilized fertilizer composition comprising siderophores of the present invention.

It will be understood by those skilled in the art that the step of separation of the microorganisms comprises several unitary operations and the corresponding equipment known to the state of the art, such as decantation and the decanter, filtration and the filters and centrifugation and the centrifuges. Similarly, it will be understood by those skilled in the art that the expression filtrate of the biotransformed medium comprises the aqueous solution of products of the process of biochemical transformation of substrates present in the nutrient medium containing siderophores, whereby the referred aqueous solution is obtained after the use of at least one unitary operation to carry out the separation of microorganisms.

In a preferred embodiment of the present invention, the preferred stabilizing agent is cornstarch. However, it will be understood by one skilled in the art that the stabilizer may be starch originating from several vegetable sources, whereby it is also within the scope of the present invention the use of mixtures of starch from several vegetable sources.

The stabilizing agent provides the production of more stable lyophilized fertilizer compositions comprising iron siderophore chelates, due to the reduction of levels of humidity by the stabilizing agent. In this manner, the lyophilized fertilizer compositions comprising siderophores according to the present invention may be presented in the form of a powder, which shows advantages in terms of facility of transport and storage, as regards an aqueous biotransformed medium with solubilized iron siderophore chelates or solubilized siderophores.

Prior to the use of the lyophilized fertilizer composition comprising iron siderophore chelates and a stabilizing agent (15) for treating plants, preferably the referred composition is dissolved in water, in a rehydration step, with a subsequent step of separation of the stabilizing agent, wherein the referred separation may be carried out, for example, by filtration or simple decantation. An additional advantage resulting from the rehydration operation is that it enables the preparation of solutions of iron siderophore chelates in higher concentrations as regards the original concentration of these compounds in the biotransformed medium, since smaller volumes of water than the ones originally present, may be used.

The separation of the stabilizing agent after the rehydration of the lyophilized fertilizer composition is preferably carried out in the use for treating plants, so that the user applies in the treatment process of the plants an aqueous solution, which does not result, for example, in clogging of the aspersion nozzles eventually used to apply the product in the plant cultivation. Additionally, the removal of the stabilizing agent, particularly the starch, would avoid that this is deposited on the plant foliage, impairing their physiological development, for example the proper functioning of the photosynthetic system.

The processes of preparation of the lyophilized fertilizer composition comprising iron siderophore chelates and a stabilizing agent (15) and of the lyophilized composition comprising siderophores and a stabilizing agent (16), according to the present invention, may be carried out in a batch mode, in a continuous mode, or in a semi-continuous mode, as will be understood by one skilled in the art, including for the step of synthesis of at least one siderophore compound by means of a biochemical transformation executed by at least one microorganism, to which corresponds the propagation of the microorganisms in an iron deficient nutrient medium, and for the step of propagation of the microorganisms in an iron enriched nutrient medium.

In an alternative embodiment of the present invention, a lyophilized composition is prepared, comprising siderophores and a stabilizing agent (16), according to the process illustrated in the embodiments of figures 3 and 4, whereby in this embodiment of lyophilized composition, the addition of the iron source, with the purpose of forming the iron siderophore chelates, is carried out preferably after the step of rehydration of the lyophilized, prior to the use in treating plants. Preferably, the iron source is added after the rehydration and the subsequent separation of the stabilizing agent, wherein the stabilizing agent may be separated by filtration or simple decantation. Alternatively, the iron source, which may be an iron salt in solid form, may be added to the lyophilized composition comprising siderophores and a stabilizing agent, being subsequently carried out the rehydration and the optional separation of the stabilizing agent, prior to the use in treating plants.

The lyophilized fertilizer composition of the present invention is particularly beneficial for treating plants cultivated in conditions of low iron availability, both for plants cultivated in soil, as for plants cultivated in hydroponic condition or for foliar application.

In the preferred embodiments of the present invention, the lyophilized fertilizer composition may be applied directly to crops cultivated in calcareous soils. In the embodiments where the cultivation is made in hydroponics, this may be free or in a substrate, wherein the substrate comprises one or more of pine bark, vermiculite, coconut fiber, rice husks, peat, gravel, volcanic rock, perlite, expanded clay and rock wool.

In the preferred embodiments of the present invention, the lyophilized fertilizer composition comprising iron siderophore chelates and a stabilizing agent, obtained from a bacterial culture of a species selected from *Azotobacter vinelandii, Bacillus megaterium, Bacillus subtilis, Pantoea allii, Pseudomonas fluorescens* and *Rhizobium radiobacter,* including their microbial consortiums, wherein the siderophores form chelates with iron cations in a pH in the range of from about 7 to about 9, preferably a pH of about 9.

In continuity, some illustrative examples of the present invention are presentd, which must not be interpreted as limitative of the invention, apart from the presentation of some comparative tests with fertilizer compositions known to the state of the art.

### Synthesis of siderophore compounds by means of the microorganism Azotobacter vinelandii

In the examples the strain *Azotobacter vinelandii* DSM 2289 was used, which was originally obtained from the collection "Deutsche Sammlung von Mikroorganismen und Zellkulturen" (DSM, Germany).

The incubation of the microorganisms was carried out in a Burk culture medium, which preparation is referenced in Newton et al. ("Direct demonstration of ammonia as an intermediate in nitrogen fixation by Azotobacter". J. Biol. Chem. 204, 445-451, 1953) and also referenced in HiMedia Laboratories "Burks Medium-M707" Mumbai, India, available at the site http://himedialabs.com/TD/M707.pdf, accessed on May 15, 2015. In the preparation of the Burk culture medium, the sucrose was replaced with glucose, in a concentration of 10 g/L. The final pH of the culture medium was adjusted to 7.0 ± 0.1.

For the propagation of the microoganisms in an iron enriched nutrient medium, iron was added in a final concentration of 29 mg/L of iron chloride (III). The propagation in an iron enriched nutrient medium was carried out at 30°C for 24 h in an orbital shaker at 150rpm. The cells, in exponential growth, were harvested by centrifugation, washed with the Burk medium without iron and suspended for incubation in an iron deficient Burk growth medium.

Subsequently, the propagation of the microorganisms was carried out in an iron deficient nutrient medium, having occurred the substantial synthesis of siderophores in this step. The propagation in an iron deficient nutrient medium was carried out at 30°C for about 72 h under 150 rpm agitation.

To carry out the qualitative determination of the production of siderophores in the synthesis step by at least one siderophore compound by means of a biochemical transformation executed by the strain *Azotobacter vinelandii* DSM 2289, the methods of Arnow and of Csaky may be used, to identify the catecolates and hydroxamates, respectively, as referenced in Payne, S.M. ("Detection, isolation, and characterization of siderophores." Methods Enzymol. 235, 329-44, 1994).

The iron deficient growth medium of the strain of *Azotobacter vinelandii* DSM 2289 exhibited positive results for the Arnow test, indicating the presence of catecol type siderophores in the filtrate obtained after the separation of microorganisms in the growth medium, having been used as positive control compound in the method the azotocheline, which is a di-catecolate. Additionally, the analysis resulted negative for the Csaky test, suggesting the absence of siderophores of the hydroxamate type, having been used as positive control compound the N-Dihydroxy-N,N'-diisopropylhexanediamide (DPH), which consists of a di-hydroxamate.

To carry out the quantitative determination of the production of siderophores in the synthesis step of at least one siderophore compound by means of a biochemical transformation executed by the strain *Azotobacter vinelandii* DSM 2289, the Chrome azurol S (CAS) method may be used, as referenced in Alexander, D.B., Zuberer, D.A.("Use of chrome azurol S reagents to evaluate siderophore production by rhizosphere bacteria" Biol. Fertil. Soils 12, 39-45, 1991). In the samples for the qualitative and/or quantitative analysis of the siderophores, the separation of the microorganisms must be carried out, for example by filtration in a filter with pore dimension of 0.45 micrometers, and the immediate storage of the filtrate of the biotransformed medium at a temperature of -20°C.

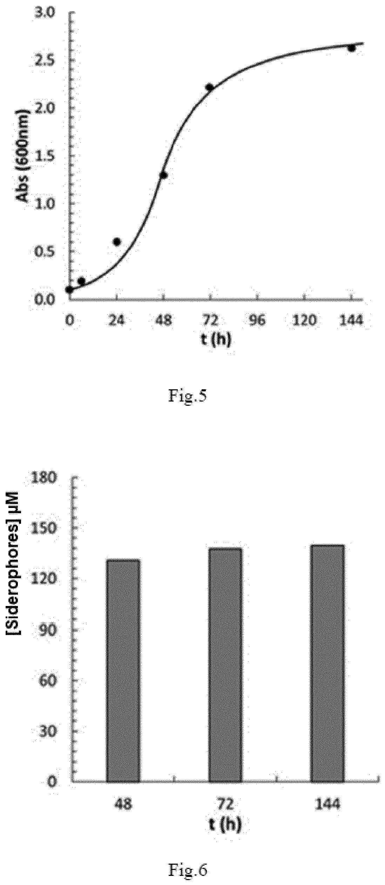

As it can be seen in Figure 5, the cell population of *A. vinelandii* reached the stationary growth stage after 72 h in the propagation step of the microorganisms in an iron deficient nutrient medium. As it can be seen in Figure 6, the siderophore concentrations, quantified by the CAS method, remain maximum and stable, at round 120 to 130 micromols equivalent of deferoxamine per liter, during the stationary growth period, from around 72 to around 144 hours) .

### Synthesis of siderophore compounds by means of other microorganisms

Additionally, the following microbial strains were used: *Bacillus megaterium* ATCC 19213, which was originally obtained from "American Type Culture Collection" (ATCC, Estados Unidos); *Bacillus subtilis* DSM 10; *Pseudomonas fluorescens* DSM 50090 and *Rhizobium radiobacter* DSM 30205.

The microorganisms were cultivated in a minimum medium with the following composition per liter: 10 g of glucose; 1.47 g of glutamic acid; 3.0 g of potassium hydrogen phosphate (K₂HPO₄); 1.0 g of potassium dihydrogen phosphate (KH₂PO₄); 0.5 g of ammonium chloride (NH₄Cl); 0.1 g of ammonium nitrate (NH₄NO₃) ; 0.1 g of sodium sulphate (Na₂SO₄) ; 10 mg of magnesium sulfate heptahydrate (MgSO₄.7H₂O); 1 mg of magnesium sulfate tetrahydrate (MnSO₄.4H₂O); and 0.5 mg of calcium chloride (CaCl₂). The final pH of the culture medium was adjusted to 7.0 ± 0.1. For the propagation of the microorganisms in an iron enriched nutrient medium, iron was added in a final concentration of 29 mg/L of iron chloride (III). Initially, the microorganisms were incubated in the minimum medium, containing iron, at a temperature of 30°C, for 8 hours, with a 150rpm agitation. Subsequently, the microorganisms were re-inoculated in the minimum medium with iron and propagated during approximately 16 h, at 30°C, with a 150rpm agitation. The cells, in exponential stage of growth, were harvested by centrifugation, washed with a minimum culture medium without iron, suspended and incubated in iron deficient minimum culture medium. The propagation of the microorganisms in iron deficient nutrient medium was made at 30°C for 48 h under a 150rpm agitation.

To carry out the qualitative determination of the siderophore production in the synthesis step of at least one siderophore compound by means of a biochemical transformation executed by the mentioned microbial strains, the Arnow and Csaky methods were used, which results are shown in Table 1. The azotocheline was used as a positive control in the Arnow method. The DPH was used as a positive control in the Csaky method.

In order to determine the potential capacity of each biotransformed medium, comprising siderophores, to complex the iron in alkalyne conditions (pH 9), a modification of the European Commettee for Standardization EN 15962 was adopted. (European Committee for Standardization. EN 15962. 2011 E. Fertilizers - determination of the complexed micronutrient content and of the complexed fraction of micronutrients. pp 14). In summary, iron chloride (III) was added to a fixed volume of a filtrate of biotransformed medium, adjusting the pH to 9.0 ± 0.1 and allowing the solution to rest for 30 minutes. Subsequently, the pH was once again adjusted and the medium was left to sediment at rest for 3 hours. The medium was subsequently centrifuged (3000xg, 10 min) and filtered in a filter with pore dimension of 0.45 micrometers. The quantity of iron in solution, herein designated soluble[Fe], was determined by atomic absorption spectrometry after the referred filtration. In continuity, a graphic representation of the soluble[Fe] versus the added[Fe], wherein the added[Fe] was calculated based on the quantity of iron chloride (III) added, was carried out. For each biotransformed medium separated from the respective microorganisms, the complexing capacity was obtained by means of the intersection of the two lines obtained, of soluble [Fe] and added[Fe], and the results are shown in Table 1.

**Table 1: summary of the main properties of the evaluated bacteria**

| Bacteria | Average complexing capacity at pH 9.0 (µmol.L⁻¹) | Type of siderophores |
|---|---|---|
| *Bacillus megaterium* | 280 | Hydroxamate |
| *Bacillus subtilis* | 225 | Catecolate |
| *Pseudomonas fluorescens* | 36 | Catecolate + Hydroxamate |
| *Rhizobium radiobacter* | 180 | Catecolate |

As can be observed from the results of Table 1, the siderophores produced by biotransformation by the cited microorganisms have the capacity to complex iron at pH 9, whereby this pH value is comprised in the typical range of the agronomic conditions in calcareous soils.

### Preparation of iron solutions

In order to provide the forming of the iron siderohore chelates in the lyophilized fertilizer composition of the present invention, Fe(NO₃)₃ was added to the filtrate originating from the medium biotransformed by *A*. *vinelandii* comprising siderophores up to a final concentration of 4.5 milimol/L of iron, the pH having been adjusted to 9.0. The solution comprising iron siderophore chelates was left to rest for some hours with no light, so as to avoid a possible photodecomposition, and was subsequently filtered in a filter with pore dimension of 0.45 micrometers, so as to remove insoluble solids, such as exceeding non-solubilized iron.

A fertilizer composition (positive control) was prepared according to the state of the art, so as to compare it with the lyophilized fertilizer composition according to the present invention. In the fertilizer composition known to the state of the art (positive control), the chelating agent used was ethylenediamine-N,N'-bis(2-hydroxyphenylacetic acid (o,o-EDDHA). In order to provide that the o,o-EDDHA forms chelates with iron cations in the comparison fertilizer composition, Fe(NO₃)₃ was added to the solution of o,o-EDDHA up to a final concentration of 4.5 milimol/L of iron and the pH of the referred solution was adjusted to 7.0. The solution of o,o-EDDHA chelate and iron was left to rest for some hours without light, so as to avoid the possible photodecomposition, and was subsequently filtered with a filter of pore dimension of 0.45 micrometers, so as to remove insoluble solids, such as exceeding non-solubilized iron.

### Response of the developments of a soybean cultivation to the treatment with iron

Comparative tests were performed with the purpose of obtaining the response to the treatment of soybean with application of a medium comprising iron in a calcareous soil. The comparative tests involved the lyophilized fertilizer composition comprising iron siderophore chelates and using cornstarch as stabilizing agent, according to the present invention, and the fertilizer composition comprising chelates formed between the o,o-EDDHA and the iron cations. The o,o-EDDHA was used as a positive control, as this compound is a synthetic chelant agent of the APCAs class, as referenced in Pinto et al. ("Biodegradable chelating agents for industrial, domestic, and agricultural applications-a review". Environ. Sci. Pollut. Res. Int. 21, 11893-906, 2014). In the comparative tests, additions were made of quantities of each of the compositions, so that each one provides 2.5 mg of iron per kg of soil.

For the soil cultivation, the starting point was seeds of the *Glycine max* cv. AG18333 soybean, which were germinated using sterile trays having perlite as substrate, as referenced in Nadal et al. ("Evaluation of Fe-N,N'-Bis(2-hydroxybenzyl)ethylenediamine-N,N'-diacetate (HBED/Fe3+) as Fe carrier for soybeanbean (Glycine max) plants grown in calcareous soil." Plant Soil 360, 349-362, 2012). The seedlings were examined, selected and transferred to a new tray, with a perforated plate floating on top of a solution of nutrients. The referred floating perforated plate is a plastic plate with small orifices, with about 0.5 cm diameter and with 1 to 2 cm space between them, which is placed, with the help of adequate support, to "float" over the surface of the nutritious solution, so that the roots are immersed in the nutritious solution. The nutrient solution comprises the following macronutrients: 1.0 mM of Ca(NO₃)₂; 0.9 mM of KNO₃; 0.3 mM of MgSO₄; and 0.1 mM of KH₂PO₄. The nutrient solution comprises the following buffered micronutrients: 2.5 µM of MnSO₄; 1.0 µM of CuSO₄; 10 µM of ZnSO₄; 1.0 µM of NiCl₂; 1.0 µM of CoSO₄; and 115.5 µM of Na₂EDTA. The nutrient solution further comprises the following micronutrients: 35 µM of NaCl; 10 µM of H₃BO₃; and 0.05 µM of Na₂MoO₄. The pH of the nutrient solution was buffered with HEPES 0.1 mM (4-(2-hydroxyethyl)-1-piperazineethanesulfonic acid)) and adjusted to 7.5 with KOH 1.0 M, the growth being maintained for 7 days.

Subsequently, the soybean shoots in chlorosis induced state, were transferred, fivefold, to pots so as to carry out comparative tests as regards the iron treatments. The pots used in the experiments were made from metacrylate cylinders externally covered with foil sheet, so as to protect the contents of the vases from the light. Additionally, a netting was placed at the bottom of the vases to permit ventilation. The pots were filled with 0.6 kg of a mixture of soil of sandy clay (70%) originating from Picassent (Valence, Spain) and calcareous sand (30%). The irrigation of the plants was carried out with a solution of nutrients with buffered pH comprising the same macronutrients previously described, with the addition of 0.1g/L of CaCO₃ and 0.1g/L of NaHCO₃, with the pH in the range of from 8.0 to 8.5.

Two treatments with iron were applied, whereby a first set of pots received a solution of o,o-EDDHA and a second set of pots received the lyophilized fertilizer composition according to the present invention. A third set of vases was used as a negative control, in which no iron chelate treatment was added. At the time of application of the treatment with the solution containing the lyophilized fertilizer according to the present invention, the application of a solution of nutrients was made, which composition was balanced taking into account the nutrients present in the treatment solution containing the lyophilized fertilizer of the present invention and which maintained substantially the same quantities of nutrients relative to the treatment carried out with the o,o-EDDHA fertilizer.

The cornstarch was removed from the lyophilized fertilizer composition according to the present invention prior to carrying out the treatment with iron.

The plants were harvested after 21 days with the treatment with iron. The leaves and roots were separated from the stem and dried in a hothouse with air entry at 60°C for 72 hours. Once dried, the different parts of the plants were weighed to determine the dry mass and subsequently calcined at 480°C. The iron concentration was quantified by spectroscope by atomic absorption, following a step of digestion with nitric acid from the resulting ashes.

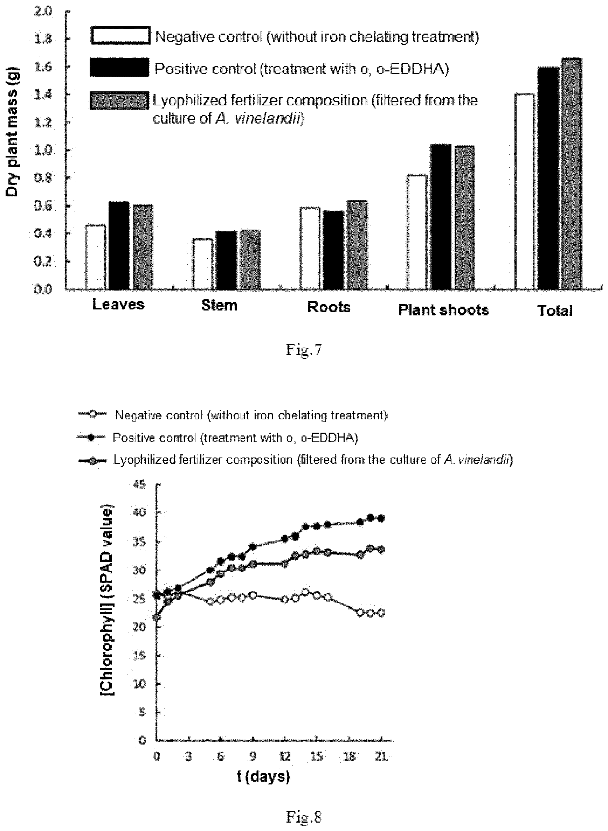

As can be observed in Figure 7, the plants treated with the composition of the present invention presentd a larger dry mass for the leaves, stems or shoots (stem and leaves), comparatively to the plants submitted to the negative control test, that is, the plants not submitted to a treatment with iron chelates. The positive effect of the treatment with the composition of the present invention is comparable to that obtained with the positive control test, that is, the plants submitted to the treatment with the solution of o,o-EDDHA and iron. Thus, while the plants of the negative control test present an average dry mass of leaves of 0.46 g, the plants treated with the composition of the present invention and the plants treated with a solution of o,o-EDDHA presentd, respectively, an average mass of leaves of 0.60 and 0.62 g. The average dry mass of the shoots was 0.82 g, 1.03 g and 1.02 g for the plants not exposed to a treatment with iron chelate (negative control), for the plants exposed to a solution of o,o-EDDHA and for the plants exposed to the composition according to the present invention, respectively.

These results demonstrate that the soybean growth was improved by the addition of the lyophilized fertilizer composition according to the present invention.

The level of plant "greenness" (SPAD value), which reflect the chlorophyll indexes in the development of plants were measured by means of a chlorophyll meter (Minolta SPAD-502; Minolta, Osaka, Japan) in all stages of the leaves, from the dycotiledons up to the last stage of the development of the plant.

The chlorophyll quantification data, collected during the experiment for the second stage of development of the leaves, which consists in the completely developed first stage after the dycotiledons of the plant can be observed in Figure 8. In the second leaf stage of the plant development, global increases of the SPAD values for the plants treated with the lyophilized fertilizer composition of the present invention were observed throughout the 21 days of the experiment, wherein the increase in the SPAD values was comparable to the increase observed in the positive control test (solution of o,o-EDDHA chelate and iron). On the contrary, the second leaf stage of the plants for the negative control test, without the treatment with iron chelate, presented lower and approximately constant SPAD values.

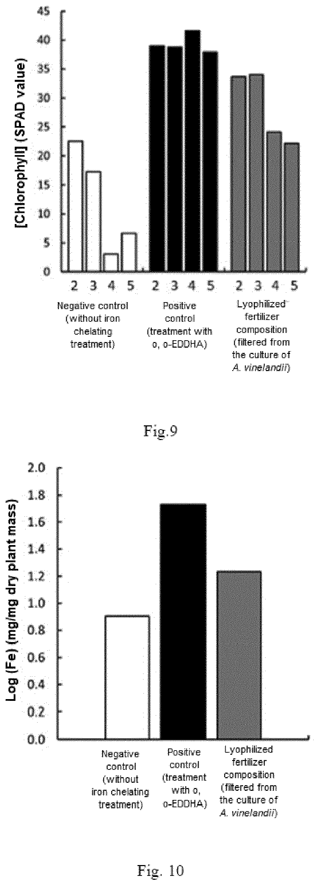

Additionally, the data referring to the quantification of chlorophyll at the end of the treatment for all leaf stages can be seen in Figure 9. It is possible to observe that the soybean plants treated with the lyophilized fertilizer composition of the present invention presented higher chlorophyll levels compared with the plants of the negative control test, that is, those that did not receive treatment with an iron chelate. The chlorophyll content in the second and third leaf stages of plants treated with the composition of the present invention was similar to that exhibited by the plants of the positive control test, that is, those treated with the solution of o,o-EDDHA and iron. Thus, the results demonstrated the positive effects of the lyophilized fertilizer composition of the present invention in the increase of the chlorophyll content of the soybean plants.

### Iron content in the plant

The quantification of iron present in the soybean leaves consists in another approach to evaluate the positive effects of the lyophilized fertilizer composition according to the present invention, as regards the treatment and prevention of plants with chlorosis.

As it can be seen in Figure 10, the lyophilized fertilizer composition according to the present invention promoted an increase in the iron content in the leaves, after 21 days of cultivation, comparatively to the plants of the negative control test, that is, those that did not receive a treatment with iron chelate.

The examples submitted demonstrate the positive effects and the ability of the lyophilized fertilizer composition according to the present invention of increasing the biomass with greener foliage in soybean cultivations in calcarous soils, wherein the vegetable biomass presents higher contents of iron and chlorophyll. In this manner, the examples submitted demonstrate that the lyophilized fertilizer compositions according to the present invention can be used in the treatment of plants with chlorosis, including those cultivated in calcareous soils.

The results in the growth curve of the microorganisms correspond to the experiments carried out twicefold, whereby each time, the growth was monitored in triplicate. All the results referring to the quantification of siderophores by CAS method were, at least, repeated twice. In the experiments conducted in the treatment of soybean with iron, each treatment was carried out fivefold. The data of Figures 5 to 10 are presented as average values.

As used in this description, the expressions "about" and "approximately" refer to a range in values of roughly 10% the specified number.

As used in this description, the expression. "substantially" means that the real value is within an interval of about 10% of the desired value, variable or related limit, particularly within about 5% of the desired value, variable or related limit or particularly within about 1% of the desired value, variable or related limit.

The subject matter described above is provided as an illustration of the present invention and must not be interpreted so as to limit it. The terminology used with the purpose of describing specific embodiments, according to the present invention, must not be interpreted to limit the invention. As used in this description, the definite and indefinite articles, in their singular form, aim to include in the interpretation the plural forms, unless the context of the description explicitly indicates the contrary. It will be understood that the expressions "comprise" and "include", when used in this description, specify the presence of the characteristics, the elements, the components, the steps and the related operations, but do not exclude the possibility of other characteristics, elements, components, steps and operations from being also contemplated.

All modifications, providing that they do not modify the essential features of the following claims, must be considered within the scope of protection of the present invention.

## Claims

1. A process for obtaining a lyophilized fertilizer composition including iron siderophores chelates **characterized by** comprising the following steps:
a) Synthesis of at least one siderophore compound by means of a biochemical transformation executed by at least one microorganism, forming a biotransformed medium comprising at least one siderophore compound;
b) Addition of an iron source to the biotransformed medium comprising at least one siderophore compound, forming an aqueous solution comprising iron siderophore chelates;
c) Addition of a stabilizing agent to the aqueous solution comprising iron siderophore chelates, forming a mixture comprising iron siderophore chelates and a stabilizing agent, wherein the stabilizing agent is starch, for example cornstarch;
d) Lyophilization of the mixture comprising iron siderophore chelates and a stabilizing agent, forming a lyophilized fertilizer composition comprising iron siderophore chelates and a stabilizing agent.

2. A process for obtaining a lyophilized fertilizer composition including iron siderophore chelates **characterized by** comprising the following steps:
a) Synthesis of at least one siderophore compound by means of a biochemical transformation executed by at least one microorganism, forming a biotransformed medium comprising at least one siderophore compound;
b) Addition of an iron source to the biotransformed medium comprising at least one siderophore compound, forming an aqueous solution comprising iron siderophore chelates;
c) Lyophilization of the aqueous solution comprising iron siderophore chelates, forming a lyophilized fertilizer composition comprising iron siderophore chelates;
d) Addition of a stabilizing agent to the lyophilized fertilizer composition comprising iron siderophore chelates, forming a lyophilized fertilizer composition comprising iron siderophore chelates and a stabilizing agent, wherein the stabilizing agent is starch, for example cornstarch.

3. A process for obtaining a lyophilized composition including siderophores **characterized by** comprising the following steps:
a) Synthesis of at least one siderophore compound by means of a biochemical transformation executed by at least one microorganism, forming a biotransformed medium comprising at least one siderophore compound;
b) Addition of a stabilizing agent to the biotransformed medium comprising at least one siderophore compound, forming a mixture comprising siderophores and a stabilizing agent, wherein the stabilizing agent is starch, for example cornstarch;
c) Lyophilization of the mixture comprising siderophores and a stabilizing agent, forming a lyophilized composition comprising siderophores and a stabilizing agent.

4. A process for obtaining a lyophilized composition including siderophores **characterized by** comprising the following steps:
a) Synthesis of at least one siderophore compound by means of a biochemical transformation executed by at least one microorganism, forming a biotransformed medium comprising at least one siderophore compound;
b) Lyophilization of the biotransformed medium comprising at least one siderophore compound, forming a lyophilized composition comprising siderophores;
c) Addition of a stabilizing agent to the lyophilized composition comprising siderophores, forming a lyophilized composition comprising siderophores and a stabilizing agent, wherein the stabilizing agent is starch, for example cornstarch.

5. The process for obtaining a lyophilized fertilizer composition including iron siderophore chelates according to any one of claims 1 and 2, **characterized by** the fact that in the addition of an iron source to the biotransformed medium step comprising at least one siderophore compound, the pH adjustment is made in the range of from about 7 to about 9.

6. The process for obtaining a lyophilized fertilizer composition including iron siderophore chelates, according to any one of claims 1, 2 and 5, **characterized by** the fact that in the addition of an iron source to the biotransformed medium comprising at least one siderophore compound step, a separation of insoluble solids is made.

7. The process for obtaining a lyophilized fertilizer composition including iron siderophore chelates, according to any one of claims 1, 2, 5 and 6, **characterized by** the fact that in the iron source addition to the biotransformed medium comprising at least one siderophore compound step, the iron source is at least one iron salt.

8. The process for obtaining a lyophilized fertilizer composition including iron siderophore chelates, according to any one of claims 1, 2, 5 to 7, or the process for obtaining a lyophilized composition including siderophores, according to any one of claims 3 and 4, **characterized by** the fact that the synthesis step of at least one siderophore compound, by means of a biochemical transformation executed by at least one microorganism, comprises:
a first propagation of the microorganisms in an iron enriched nutrient medium; and
a second propagation of the microorganisms in an iron deficient nutrient medium.

9. The process for obtaining a lyophilized fertilizer composition including iron siderophore chelates, according to any one of claims 1, 2, 5 to 8, or the process for obtaining a lyophilized composition including siderophores, according to any one of claims 3, 4 and 8, **characterized by** the fact that the biotransformed medium, originating from the synthesis of at least one siderophore compound by means of a biochemical transformation executed by at least one microorganism, comprises the filtrate of the step of the second propagation of microorganisms in an iron deficient nutrient medium.

10. The process for obtaining a lyophilized fertilizer composition including iron siderophore chelates, according to any one of claims 1, 2, 5 to 9, or the process of obtaining a lyophilized composition including siderophores, according to any one of claims 3, 4, 8 and 9, **characterized by** the fact that the synthesis step of at least one siderophore compound by means of a biochemical transformation executed by at least one microorganism comprises a step of separation of the microorganisms after the synthesis of the siderophores.

11. The process for obtaining a lyophilized fertilizer composition including iron siderophore chelates, according to any one of claims 1, 2, 5 to 10, or the process of obtaining a lyophilized composition including siderophores, according to any one of claims 3, 4, 8 to 10, **characterized by** the fact that in the synthesis step of at least one siderophore compound by means of a biochemical transformation, the microorganism is selected from one or more of the genus *Azotobacter, Azospirillum, Bacillus, Enterobacter, Nocardia, Pantoea, Paracoccus, Pseudomonas, Rhizobium, Salinispora, Streptomyces* and *Vibrio.*

12. A lyophilized fertilizer composition, **characterized by** comprising:
iron siderophore chelates; and
a stabilizing agent, wherein the stabilizing agent is starch, for example cornstarch.

13. The lyophilized fertilizer composition according to the previous claim, **characterized by** the fact that it is obtained from the process for obtaining a lyophilized fertilizer composition including iron siderophore chelates, as defined in any one of claims 1, 2 and 5 to 11.

14. A lyophilized composition, **characterized by** comprising:
siderophores; and
a stabilizing agent, wherein the stabilizing agent is starch, for example cornstarch.

15. The lyophilized composition, according to the previous claim, **characterized by** being obtained from the process for obtaining a lyophilized composition including siderophores as defined in any one of claims 3, 4, and 8 to 11.

16. A use of a lyophilized fertilizer composition including iron siderophore chelates for treating plants, **characterized by** the use of a lyophilized fertilizer composition including iron siderophore chelates, as defined in any one of claims 12 and 13.

17. A use of a lyophilized fertilizer composition including iron siderophore chelates for treating plants, **characterized by** the use of a lyophilized composition including siderophores, as defined in any one of claims 14 and 15, and by the addition of an iron source to the said lyophilized composition including siderophores.

18. The use of a lyophilized fertilizer composition including iron siderophore chelates for treating plants, according to claim 16, or the use of a lyophilized composition including siderophores for treating plants, according to claim 17, **characterized by** the fact that the plants are cultivated in conditions with low iron availability.

19. The use of a lyophilized fertilizer composition including iron siderophore chelates for treating plants, according to any one of claims 16 to 18, or the use of a lyophilized composition including siderophores for treating plants, according to any one of claims 17 and 18, **characterized by** the fact that the plants are cultivated by foliar nutrition or hydroponics, free or in a substrate, wherein the substrate comprises one or more of pine bark, vermiculite, coconut fiber, rice husks, peat, gravel, volcanic rock, perlite, expanded clay, and rock wool.

20. The use of a lyophilized fertilizer composition including iron siderophore chelates for treating plants, according to any one of claims 16 and 18, or the use of a lyophilized composition including siderophores for treating plants, according to any one of claims 17 and 18, **characterized by** the fact that the plants are cultivated in soil.

21. The use of a lyophilized fertilizer composition including iron siderophore chelates for treating plants or the use of a lyophilized composition including siderophores for treating plants, according to claim 20, **characterized by** the fact that the soil is calcareous.

22. The use of a lyophilized fertilizer composition including iron siderophore chelates for treating plants, according to any one of claims 16 and 18 to 21, or the use of a lyophilized composition including siderophores for treating plants, according to any one of claims 17 and 18 to 21, **characterized by** the fact that the lyophilized fertilizer composition including iron siderophore chelates or the lyophilized composition including siderophores is rehydrated and the stabilizing agent is optionally separated prior to the use for treating plants.
